(19) Europäisches Patentamt
European Patent Office
Office européen des brevets

(11) Veröffentlichungsnummer: **0 270 964 B1**

(12) **EUROPÄISCHE PATENTSCHRIFT**

(45) Veröffentlichungstag der Patentschrift: **08.07.92**

(51) Int. Cl.$^5$: **C07C 205/11**, C07C 255/50, C07C 201/16, C07C 51/64, C07C 63/10

(21) Anmeldenummer: **87117653.3**

(22) Anmeldetag: **28.11.87**

(54) **Verfahren zur Gewinnung von substituierten Fluorbenzolen.**

(30) Priorität: **11.12.86 DE 3642326**

(43) Veröffentlichungstag der Anmeldung:
**15.06.88 Patentblatt 88/24**

(45) Bekanntmachung des Hinweises auf die Patenterteilung:
**08.07.92 Patentblatt 92/28**

(84) Benannte Vertragsstaaten:
**CH DE FR GB IT LI**

(56) Entgegenhaltungen:
**DE-A- 3 400 418**
**DE-A- 3 435 889**
**US-A- 3 294 629**

**HYDROCARBON PROCESSING, Band 47, Nr. 9, September 1968 "DMSO extraction" Seite 177**

(73) Patentinhaber: **BAYER AG**

**W-5090 Leverkusen 1 Bayerwerk(DE)**

(72) Erfinder: **Blank, Heinz Ulrich, Dr.**
**Am Geusfelde 35**
**W-5068 Odenthal(DE)**
Erfinder: **Ritzer, Edwin, Dr.**
**Burbachstrasse 31**
**W-5093 Burscheid(DE)**

**EP 0 270 964 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft ein Verfahren zur Gewinnung von substituierten Fluorbenzolen aus aprotischen, polaren Lösungsmitteln bzw. solche Lösungsmittel enthaltenden Gemischen, das dadurch gekennzeichnet ist, daß man die substituierten Fluorbenzole mit aliphatischen Extraktionsmitteln aus den Lösungsmitteln/Lösungsmittelgemischen extrahiert und danach die Extraktionsmittel von den substituierten Fluorbenzolen abtrennt oder die substituierten Fluorbenzole in den Extraktionsmitteln einer chemischen Umsetzung unterzieht und die Extraktionsmittel von den Umsetzungsprodukten der substituierten Fluorbenzole abtrennt.

Bei der Herstellung substituierter Fluorbenzole fallen diese häufig in aprotischen, polaren Lösungsmitteln an bzw. in Gemischen, die aprotische, polare Lösungsmittel enthalten. Beispiele für Herstellungsverfahren, die zu solchen Lösungen führen, sind nucleophile Austauschreaktionen, wie die in US 3.294.629 beschriebene Herstellung von 3,5-Dichlor-2,4-difluor-nitrobenzol aus 2,3,4,5-Tetrachlor-nitrobenzol und Kaliumfluorid in Dimethylsulfoxid (DMSO).

Die Isolierung der stark polaren substituierten Fluorbenzole aus den ebenfalls stark polaren aprotischen Lösungsmitteln stößt auf erhebliche Schwierigkeiten wegen der hohen Reaktivität und thermischen Empfindlichkeit der zu isolierenden Substanzen aufgrund ihrer häufig vielfältigen Substitution und weiterhin auf Schwierigkeiten wegen der durchweg hohen Siedepunkte der infrage kommenden aprotischen, polaren Lösungsmittel.

So ist eine direkte destillative Gewinnung substituierter Fluorbenzole ohne zu lange thermische Belastung nur möglich, wenn das substituierte Fluorbenzol einen tieferen Siedepunkt als das im Rahmen seiner Herstellung benutzte Lösungsmittel hat. Hat das Lösungsmittel einen tieferen Siedepunkt als das substituierte Fluorbenzol, muß zunächst unter thermischer Belastung des gewünschten Produktes das Lösungsmittel abdestilliert werden. Im allgemeinen sind danach zur weiteren Aufarbeitung des Destillationsrückstandes mit dem gewünschten Produkt verschiedene Wasch- und Reinigungsvorgänge nötig (EP 52 833). Für den Fall, daß die Siedepunkte des substituierten Fluorbenzols und des Lösungsmittels gleich oder recht ähnlich sind oder daß sich Azeotrope bilden, ist eine direkte Destillation auch mit hohem apparativen Aufwand nicht in zufriedenstellendem Maß möglich.

Zur Vermeidung der bei hohen Destillationstemperaturen zu befürchtenden Zersetzungen und Nebenreaktionen ist vielfach, so auch in der obengenannten US 3.294.629, der Weg beschritten worden, das nach der nucleophilen Substitution in einem aprotischen, polaren Lösungsmittel vorliegende Reaktionsgemisch mit einer, bezogen auf die Menge dieses Lösungsmittels, überschüssigen Menge Wasser zu vermischen. Hierbei fällt das substituierte Fluorbenzol aus und scheidet sich als Feststoff oder als ölige Phase ab. Zur weiteren Aufarbeitung und Reinigung wird sodann das abgeschiedene substituierte Fluorbenzol in einem polaren Lösungsmittel aufgenommen.

Der Vorteil von polaren, aprotischen Lösungsmitteln bei nucleophilen Austauschreaktionen an Aromaten ist schon lange bekannt, weil beispielsweise hierdurch die Reaktion bei tieferen Temperaturen als ohne Verwendung eines Lösungsmittels durchgeführt werden kann. Da diese Lösungsmittel aprotisch sein müssen, dürfen sie auch kein Wasser enthalten. Um die polaren, aprotischen Lösungsmittel wiederzugewinnen, muß daher nach einer wäßrigen Aufarbeitung, wie oben beschrieben, das Wasser-Lösungsmittel-Gemisch destillativ aufgearbeitet werden. Da das Wasser im Vergleich zu den polaren, aprotischen Lösungsmitteln im allgemeinen den tieferen Siedepunkt hat, muß man es zuerst abdestillieren und kann erst anschließend das Lösungsmittel gewinnen. Diese Destillation ist besonders deshalb sehr energieaufwendig, weil das Wasser in einem großen Überschuß vorliegt, da bei der oben beschriebenen wäßrigen Aufarbeitung eine möglichst vollständige Ausfällung des zu gewinnenden substituierten Fluorbenzols erfolgen soll und weiterhin bei ungenügendem Wasserzusatz die Phasentrennung zwischen dem öligen substituierten Fluorbenzol und dem Wasser-Lösungsmittel-Gemisch mangelhaft ist. Im Anschluß an die destillative Trennung von Wasser und Lösungsmittel folgt noch ein mehr oder minder aufwendig durchzuführendes Absolutierungsverfahren für das Lösungsmittel vor seinem Wiedereinsatz. Auch beim Versuch, das substituierte Fluorbenzol durch Wasserdampfdestillation aus dem Lösungsmittel zu gewinnen (US 4.140.719), ergeben sich ähnliche Schwierigkeiten bezüglich der Rückgewinnung des Lösungsmittels.

Die extraktive Gewinnung von Benzol, Toluol und Xylol aus Kohlenwasserstoffen unter Benutzung von Dimethylsulfoxid (DMSO), N-Methyl-pyrrolidon (NMP), Ethylenglykol und anderen polaren Extraktionsmedien ist bekannt (DE-AS 12 69 272; Erdöl und Kohle-Erdgas-Petrochemie 21 (1968), 275). Das Extraktionsmittel ist in diesem Falle die stärker polare Substanz gegenüber dem als Lösungsmittel anzusehenden restlichen Kohlenwasserstoff-Gemisch. Hierbei ist auch der Zusatz einer kleinen Wassermenge zum Extraktionsmittel vorgeschlagen worden, wodurch dessen Selektivität erhöht werden kann, gleichzeitig aber die Kapazität für die genannten Aromaten sinkt. Bei den einfachen und wenig polaren Aromaten Benzol,

Toluol und Xylol ist auch eine Rückgewinnung aus dem Gemisch mit DMSO durch Extraktion mit Paraffinen möglich, wenn dem DMSO Wassermengen von bis zu 15 % zugesetzt werden (Erdöl und Kohle-Erdgas-Petrochemie, loc. cit.; Hydrocarbon Processing 47 (September 1968), 177 und 51 (September 1972), 185).

Angesichts dieser Zusammenhänge ist es überraschend, daß hochpolare, substituierte Fluorbenzole mit nahezu unpolaren aliphatischen Extraktionsmitteln aus polaren aprotischen Lösungsmitteln extrahiert werden können. Diese Extraktion ist selbst dann möglich, wenn diesen aprotischen, polaren Lösungsmitteln kein Wasser zugesetzt wurde.

Substituierte Fluorbenzole, die im erfindungsgemäßen Verfahren gewonnen werden können, sind solche der allgemeinen Formel

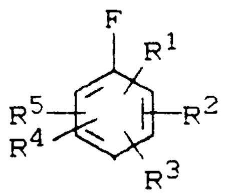 (I),

in der

- $R^1$ für eine zum Fluor ortho- oder para-ständige $NO_2$-, CN-, COF- oder $CF_3$-Gruppe steht und für den Fall, daß mindestens drei der Reste $R^2$ bis $R^5$ Chlor oder Brom darstellen, von denen eines zum Fluor ortho-ständig ist, auch das zweite ortho-ständige Chlor oder Brom sein kann,
- $R^2$ Wasserstoff, Halogen, eine $NO_2$-, CN-, COF-, $CF_3$-, $COOR^6$-, $COR^7$-, $SO_2R^7$-, $SO_2$-$N(R^6)_2$- oder CO-$N(R^6)_2$-Gruppe bedeutet, worin
  $R^6$ Alkyl oder Phenyl und
  $R^7$ Alkyl, Phenyl oder substituiertes Phenyl bedeuten,
- $R^3$ Wasserstoff, Halogen, Alkyl oder eine $NO_2$-Gruppe bedeutet und
- $R^4$ und $R^5$ unabhängig voneinander Wasserstoff oder Halogen bedeuten.

Halogen ist beispielsweise Fluor, Chlor oder Brom, bevorzugt Fluor oder Chlor.

Alkyl hat beispielsweise 1-6, bevorzugt 1-4, besonders bevorzugt 1-2 C-Atome, wie Methyl, Ethyl, Propyl, Isopropyl, Butyl, Isobutyl, Amyl, Hexyl. Ganz besonders bevorzugt ist Methyl.

Für den Fall eines substituierten Phenyl kommen ein oder mehrere der Substituenten in Frage, die unter $R^1$ und $R^2$ genannt sind. Sie können gleiche oder verschieden sein.

Unter die Formel (I) fallende Stoffe sind beispielsweise: Nitrofluorbenzole, wie z.B. 2-Fluornitrobenzol, 4-Fluornitrobenzol, 2,4-Difluornitrobenzol, 2,6-Difluornitrobenzol, 2,4,6-Trifluornitrobenzol; Nitrochlorfluorbenzole, wie z.B. 3-Chlor-4-fluornitrobenzol, 3-Chlor-2-fluornitrobenzol, 5-Chlor-2-fluornitrobenzol, 3-Chlor-2,4-difluornitrobenzol, 5-Chlor-2,4-difluornitrobenzol, 3,5-Dichlor-2-fluornitrobenzol, 3,5-Dichlor-4-fluornitrobenzol, 3,5-Dichlor-2,4-difluornitrobenzol; Nitrofluortoluole,wie z.B. 2-Fluor-5-nitrotoluol, 3-Fluor-6-nitrotoluol, 2-Fluor-3-nitrotoluol, 3-Fluor-4-nitrotoluol, 4-Fluor-3-nitrotoluol, 3-Fluor-2-nitrotoluol; Dinitrofluorbenzole, wie z.B. 2,4-Dinitro-fluorbenzol, 2,6-Dinitrofluorbenzol, 2,3-Dinitro-fluorbenzol, 3,4-Dinitro-fluorbenzol, 2,5-Dinitro-fluorbenzol; Dinitrochlorfluorbenzole, wie z.B. 6-Chlor-2,4-dinitro-fluorbenzol, 4-Chlor-2,6-dinitro-fluorbenzol; Fluorbenzonitrile, wie z.B. 4-Fluorbenzonitril, 2-Fluorbenzonitril, 2,4-Difluorbenzonitril, 2,6-Difluorbenzonitril, 3,5-Difluorbenzonitril, 3,4-Difluorbenzonitril, 2,3-Difluorbenzonitril, 2,4,6-Trifluorbenzonitril, 2,3,4-Trifluorbenzonitril, 2,4,6-Trifluorbenzonitril, 2,3,5-Trifluorbenzonitril, 3,4,5-Trifluorbenzonitril, 2,3,6-Trifluorbenzonitril, 2,3,4,5-Tetrafluorbenzonitril, 2,3,4,6-Tetrafluorbenzonitril, 2,3,5,6-Tetrafluorbenzonitril, Pentafluorbenzonitril; Fluorbenzoylfluoride, wie z.B. 4-Fluorbenzoylfluorid, 2-Fluorbenzoylfluorid, 2,4-Difluorbenzoylfluorid, 2,6-Difluorbenzoylfluorid, 3,5-Difluorbenzoylfluorid, 3,4-Difluorbenzoylfluorid, 2,3-Difluorbenzoylfluorid, 2,4,6-Trifluorbenzoylfluorid, 2,3,4-Trifluorbenzoylfluorid, 2,4,6-Trifluorbenzoylfluorid, 2,3,5-Trifluorbenzoylfluorid, 3,4,5-Trifluorbenzoylfluorid, 2,3,6-Trifluorbenzoylfluorid, 2,3,4,5-Tetrafluorbenzoylfluorid, 2,3,4,6-Tetrafluorbenzoylfluorid, 2,3,5,6-Tetrafluorbenzoylfluorid, Pentafluorbenzoylfluorid; Fluor-trifluormethyl-benzole, wie z.B. 4-Fluortrifluormethyl-benzol, 2-Fluor-trifluormethyl-benzol, 2,4-Difluor-trifluormethyl-benzol, 2,6-Difluor-trifluormethyl-benzol, 3,5-Difluor-trifluormethyl-benzol, 3,4-Difluor-trifluormethyl-benzol, 2,3-Difluor-trifluormethyl-benzol, 2,4,6-Trifluor-trifluormethyl-benzol, 2,3,5-Trifluor-trifluormethyl-benzol, 2,4,5-Trifluortrifluormethyl-benzol, 2,3,6-Trifluor-trifluormethylbenzol, 2,3,4,5-Tetrafluor-trifluormethyl-benzol, 2,3,4,6-Tetrafluor-trifluormethyl-benzol, 2,3,5,6-Tetrafluor-trifluormethyl-benzol, Pentafluor-trifluormethyl-benzol; Chlorfluor-benzonitrile, wie z.B. 3-Chlor-4-fluorbenzonitril, 3-Chlor-2-fluorbenzonitril, 3-Chlor-6-fluorbenzonitril, 3,5-Dichlor-4-fluorbenzonitril, 3,5-Dichlor-2-fluorbenzonitril, 3-Chlor-2,6-difluorbenzonitril, 3-Chlor-2,4-difluorbenzonitril, 5-Chlor-2,4-difluorbenzonitril, 5-Chlor-3,4-difluorbenzonitril, 3,5-Dichlor-2,4-difluorbenzonitril, 3,5-Dichlor2,6-difluorbenzonitril, 3,5-Dichlor-2,4,6-trifluorbenzonitril; Chlorfluor-benzoylfluoride wie z.B. 3-Chlor-4-fluor-benzoylfluorid, 3-Chlor-2-fluor-benzoylfluorid, 3-Chlor-6-fluor-benzoylfluorid, 3,5-Dichlor-4-fluor-benzoylfluorid, 3,5-Dichlor-2-fluor-benzoylfluorid, 3-Chlor-2,6-difluor-benzoylfluorid, 3-Chlor-2,4-difluorbenzoylfluorid, 5-Chlor-2,4-difluor-benzoylfluorid, 5-Chlor-3,4-difluor-benzoylfluorid, 3,5-Dichlor-2,4-difluor-benzoylfluorid, 3,5-Dichlor-2,6-difluor-benzoylfluorid, 3,5-Dichlor-2,4,6-trifluor-benzoylfluorid, Chlorfluor-trifluormethyl-benzole, wie z.B. 3-Chlor-4-fluor-trifluormethyl-benzol, 3-Chlor-2-fluor-trifluormethyl-benzol, 3-Chlor-6-fluor-trifluormethyl-benzol, 3,5-Dichlor-4-fluor-trifluormethyl-benzol, 3,5-Dichlor-2-fluor-trifluormethyl-benzol, 3-Chlor-2,6-difluortrifluormethyl-benzol, 3-Chlor-2,4-difluor-trifluormethyl-benzol, 5-Chlor-2,4-difluor-trifluormethylbenzol, 5-Chlor-3,4-difluor-trifluormethyl-benzol, 3,5-Dichlor-2,4-difluor-trifluormethyl-benzol, 3,5-Dichlor2,6-difluor-trifluormethyl-benzol, 3,5-Dichlor-2,4,6-trifluor-trifluormethyl-benzol; Fluor-chlorbenzole, wie z.B. 2,3,5,6-Tetrachlor-fluorbenzol, 2,3,4,6-Tetrachlorfluorbenzol; Brom-nitrofluor-benzole, wie z.B. 5-Brom-2-fluor-nitrobenzol; 4-Fluor-3-nitrobenzoesäureester, wie z.B. 4-Fluor-3-nitrobenzoesäuremethylester; Nitrotrifluormethyl-fluorbenzole, wie z.B. 4-Fluor-3-trifluormethyl-nitrobenzol, 2-Fluor-5-trifluormethyl-nitrobenzol, 2,6-Dinitro-4-trifluormethyl-fluorbenzol; Halogen-benzophenone, wie z.B. 4,4′-Difluor-benzophenon; Halogen-diphenylsulfone, wie z.B. 4,4′-Difluor-diphenylsulfon.

Stoffe der genannten Art können durch nucleophilen Halogen-Fluor-Austausch in aprotischen, polaren Lösungsmitteln erhalten werden. Bei Tetra- oder Penta-chlor(brom)-nitrobenzolen mit beiden zur Nitrogruppe orthoständigen durch Chlor(Brom) besetzten Positionen findet auch ein Nitro-Fluor-Austausch zum entsprechenden Tetra-oder Penta-chlor(brom)-fluorbenzol statt.

Stoffe der genannten Art können auch als Gemisch im aprotischen, polaren Lösungsmittel vorliegen, beispielsweise das aus US 3.294.629 bekannte Isomerengemisch aus 84 % 2,5-Dichlor-4-fluor-nitrobenzol und 16 % 4,5-Dichlor-2-fluor-nitrobenzol. Für den Fall, daß Stoffe der genannten Art nicht aus reinen Vorstufen sondern von solchen technischer Qualität hergestellt werden, werden auch die meist chemisch ähnlichen Begleitstoffe, sowie unvollständig umgesetzte Ausgangsstoffe und Nebenprodukte erfindungsgemäß gewonnen.

Bevorzugte substituierte Fluorbenzole, die erfindungsgemäß gewonnen werden können, sind gegebenenfalls substituierte Fluor-nitrobenzole der Formel

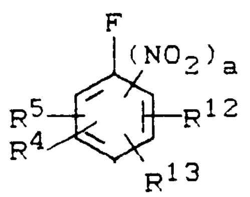

(II),

in der

a die ortho- oder para-Position anzeigt,

$R^{12}$ und $R^{13}$ unabhängig voneinander Wasserstoff, die $NO_2$-oder $CF_3$-Gruppe oder Halogen bedeuten, wobei $R^{13}$ zusätzlich noch Alkyl bedeuten kann, und

$R^4$ und $R^5$ unabhängig voneinander Wasserstoff oder Halogen bedeuten.

Besonders bevorzugte Fluorbenzole, die erfindungsgemäß gewonnen werden können, sind substituierte Fluor-nitrobenzole der Formel

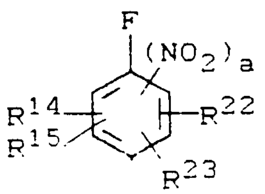

(III),

in der

a die ortho- oder para-Position anzeigt,

$R^{22}$ und $R^{23}$ unabhängig voneinander die $NO_2$- oder $CF_3$-Gruppe, Fluor oder Chlor bedeuten, wobei

$R^{23}$ zusätzlich noch Alkyl bedeuten kann, und
$R^{14}$ und $R^{15}$ unabhängig voneinander Wasserstoff, Fluor oder Chlor bedeuten.

Aprotische, polare Lösungsmittel im Rahmen des erfindungsgemäßen Verfahrens sind beispielsweise Dimethylsulfoxid (DMSO), Dimethylsulfon ($DMSO_2$), Dimethylformamid (DMF), Acetonitril, Tetramethylensulfon (Sulfolan; $TMSO_2$), Dimethylacetamid (DMA), Hexamethyl-phosphorsäuretriamid, bevorzugt DMSO, $DMSO_2$, DMF, DMA, Acetonitril oder $TMSO_2$, besonders bevorzugt DMSO, DMF oder $TMSO_2$.

Aliphatische Extraktionsmittel für das erfindungsgemäße Verfahren sind geradkettige oder verzweigte, offenkettige oder cyclische aliphatische Kohlenwasserstoffe, deren Siedepunkt mindestens 30 °C beträgt, wie Pentan, Hexan, Octan, Isooctan, Decan, Dodecan, Isododecan, Hexadodecan, Cyclohexan, Methylcyclopentan, Methylcyclohexan, Decalin, Cyclooctan, Ethyl-cyclohexan, sowie die aliphatischen Destillationsschnitte Petrolether mit Siedebereichen 30-50 ° C, ca. 40 ° C, 40-60 ° C, 60-70 ° C, 40-80 ° C, Leichtbenzin (60-95 ° C), Ligroin (80-110 ° C), Lötbenzin (60-140 ° C), Waschbenzin (100-140 ° C) und andere, sowie deren Gemische untereinander. In bevorzugter Weise werden aliphatische Extraktionsmittel mit einem Siedepunkt von mindestens 60 ° C eingesetzt, beispielsweise Hexan, Octan, Isooctan, Dodecan, Isododecan, Leichtbenzin, Ligroin, Lötbenzin, Decalin, Cyclooctan, Cyclohexan, Methyl-cyclohexan, Ethyl-cyclohexan oder Waschbenzin.

Das erfindungsgemäße Verfahren wird bei einer Temperatur von -20 bis 160 ° C, bevorzugt von -10 bis 100 ° C, besonders bevorzugt von 0 bis 60 ° C, durchgeführt.

Das erfindungsgemäße Verfahren kann grundsätzlich in diskontinuierlichen Extraktionsschritten oder kontinuierlich in dem Fachmann hierfür bekannten Extraktionsapparaten durchgeführt werden, die für eine Extraktion mit spezifisch leichteren Extraktionsmitteln ausgelegt sind. Pro Extraktionsschritt verwendet man 10-400 ml, bevorzugt 40-200 ml, besonders bevorzugt 80-140 ml, Extraktionsmittel pro 100 ml der zu extrahierenden Lösung im polaren, aprotischen Lösungsmittel. Der Fortschritt der Extraktion kann in bekannter Weise analytisch verfolgt werden; die Zahl der bei einer gewerblichen Durchführung erforderlichen Extraktionsstufen ist nach einfachen Vorversuchen eine feststehende Größe. Für den Fall einer kontinuierlichen Durchführung kann die weiter unten beschriebene anschließende Aufarbeitung des Extraktes soweit gleichzeitig durchgeführt werden, daß ständig Extraktionsmittel für den ersten Schritt des erfindungsgemäßen Verfahrens zur Verfügung steht. In einem solchen Fall ist die Menge des eingesetzten Extraktionsmittels nur von wirtschaftlichen Überlegungen bezüglich der Rückführung dieses Extraktionsmittels abhängig.

Wenn die substituierten Fluorbenzole in einem aprotischen, polaren Lösungsmittel oder einem ein solches Lösungsmittel enthaltendem Gemisch aus einer nucleophilen Halogen-Fluor-Austauschreaktion anfallen, kann die erfindungsgemäße Extraktion vor oder nach der Abtrennung der Salze (z.B. KF/KCl), sowie auch in Gegenwart von etwa benutzten Phasentransferkatalysatoren ohne Beeinträchtigung durchgeführt werden.

Das erfindungsgemäße Verfahren kann mit oder ohne Zusatz von Wasser zu den die substituierten Fluorbenzole enthaltenden aprotischen, polaren Lösungsmitteln bzw. solche Lösungsmittel enthaltenden Gemischen durchgeführt werden. Für den Fall, daß die zu extrahierende Lösung vor der Extraktion Wasser enthält, sei beispielsweise ein Gehalt von 0,1-15 Gew.-%, bezogen auf die Menge des Lösungsmittels, genannt. Es ist jedoch bevorzugt, das erfindungsgemäße Verfahren ohne Zugabe von Wasser unter weitgehendem Ausschluß von Feuchtigkeit durchzuführen. Dadurch ist das nach dem Extraktionsschritt zurückbleibende aprotische, polare Lösungsmittel bzw. das solche Lösungsmittel enthaltende Gemisch entweder sofort in eine nucleophile Austauschreaktion einsetzbar oder es bedarf hierzu nur einer wenig aufwendigen Entfernung etwaiger Spuren von Feuchtigkeit.

Das ein aprotisches, polares Lösungsmittel enthaltende Gemisch kann entweder ein Gemisch verschiedener solcher aprotischer, polarer Lösungsmittel sein oder andereinerte Lösungsmittel in einer Menge von bis zu 50 Gew.-%, bezogen auf das Gesamtgewicht des aprotischen, polaren Lösungsmittels und des anderen inerten Lösungsmittelbestandteils, enthalten, wie Benzol, Toluol, Chlorbenzol oder Dichlorbenzol.

Im Rahmen des erfindungsgemäßen Verfahrens kann das Extraktionsmittel von den substituierten Fluorbenzolen abgetrennt werden. Dies kann beispielsweise so durchgeführt werden, daß das Extraktionsmittel zum Teil abgedampft wird, bis eine Kristallisation des substituierten Fluorbenzols eintritt. Das Extraktionsmittel kann jedoch auch so gewählt werden, daß sein Siedepunkt deutlich unter dem der substituierten Fluorbenzole liegt. In einem solchen Fall kann zunächst das gesamte Extraktionsmittel abdestilliert werden, wonach entweder die substituierten Fluorbenzole durch schonende Destillation, durch Umkristallisation oder durch Säulenchromatographie des Destillationsrückstandes oder durch andere geeignete Methoden gewonnen werden. Eine destillative Abtrennung des Extraktionsmittels ist bevorzugt.

Es ist jedoch möglich, die Extraktionslösung für anschließende chemische Umsetzungen direkt einzusetzen und das Extraktionsmittel von den Umsetzungsprodukten der substituierten Fluorbenzole abzutrennen. So kann im Falle eines gegebenenfalls substituierten Fluor-nitrobenzols anschließend eine Hydrierung zum entsprechenden Anilin vorgenommen werden. Hierzu wird die Extraktioslösung, gegebenenfalls nach Zwischenschaltung eines wäßrigen Waschschrittes, eingesetzt und nach Zusatz eines geeigneten Hydrierkatalysators katalytisch mit $H_2$ hydriert. Die Wahl des aliphatischen Extraktiosmittels kann daher schon im Hinblick auf die anschließende Umsetzung erfolgen.

Das erfindungsgemäße Verfahren hat folgende Vorteile:

1) Durch die erfindungsgemäße Extraktion kann man zum Zweck der Produktgewinnung und -aufarbeitung weniger stabile Lösungsmittel verlassen und kann mit temperatur- und destillationsstabilen Lösungsmitteln weiterarbeiten.

2) Für den Fall, daß das verwendete aprotische, polare Lösungsmittel und das zu gewinnende substituierte Fluorbenzol den gleichen oder einen ähnlichen Siedepunkt haben, kann durch geeignete Auswahl des Extraktionsmittels ein durch Destillation auftrennbares Gemisch erhalten werden.

3) Gegenüber der oben beschriebenen wäßrigen Aufarbeitung von Lösungen von substituierten Fluorbenzolen in aprotischen, polaren Lösungsmitteln, bei denen das Lösungsmitteln mittels Wasser aus dem Produkt extrahiert wird, bringt die erfindungsgemäße umgekehrte Verfahrensweise eine einwandfreie Phasentrennung.

4) Das erfindungsgemäße Verfahren ist auch in Gegenwart der obengenannten Mengen Wasser in der Lösung des aprotischen, polaren Lösungsmittels durchführbar, wenngleich dies die weniger bevorzugte Verfahrensvariante darstellt. Durch den zusatz an Wasser ist jedoch die Extraktionszeit zu verkürzen, der Nachteil dieser weniger bevorzugten Verfahrensvariante ist jedoch die bereits genannte Notwendigkeit, das aprotische, polare Lösungsmittel wieder zu entwässern.

5) Die bevorzugte Verfahrensvariante ohne Gegenwart von Wasser ist in ihrer Durchführbarkeit (Extraktion von polaren Substanzen aus polaren Lösungsmitteln mit Hilfe von unpolaren Extraktionsmitteln) sehr überraschend, da das Lösungsmittel, beispielsweise DMSO, in absoluter, wasserfreier Form eine ungleich höhere Kapazität für Aromaten aufweist als in Anwesenheit von z.B. 10 Gew.-% Wasser. Darüber hinaus erlaubt es die wasserfreie Rückgewinnung des aprotischen, polaren Lösungsmittels ohne zusätzliche aufwendige Absolutierungsvorgänge.

6) Bei der Auswahl des aprotischen, polaren Lösungsmittels, beispielsweise für eine nucleophile Austauschreaktion kann der Aspekt des Siedepunktes dieses Lösungsmittels unberücksichtigt bleiben, weil durch die erfindungsgemäße Extraktion auf ein System übergegangen werden kann, das einen gewünschten Siedepunktunterschied aufweist.

7) Die restlose Abtrennung des Extraktionsmittels von dem vorgelegten aprotischen, polaren Lösungsmittel ist nicht besonders kritisch, da die Extraktionsmittel im Gegensatz zu Wasser die nucleophile Substitution nicht verhindern und somit für den Einsatz des aprotischen, polaren Lösungsmittels in einer solchen nucleophilen Substitution geringe Anteile an Extraktionsmittel toleriert werden können. Hierdurch ist bei einer etwa notwendig werdenden nachträglichen Abtrennung von Extraktionsmittel vom aprotischen, polaren Lösungsmittel eine Destillation ohne Kolonne im allgemeinen hinreichend gut.

8) Durch die Möglichkeit, das erfindungsgemäße Verfahren unter Ausschluß von Wasser erfolgreich durchzuführen, ist es nicht nur möglich, thermisch labile Verbindungen zu isolieren, sondern es ist überhaupt erst möglich, auch hydrolisierbare Verbindungen aus solchen Lösungen zu isolieren.

Beispiel 1

Zur Extraktion im Labormaßstab wurde ein Rotationsperforator zur Flüssig-Flüssig-Extraktion nach Ludwig (DE-AS 22 21 554) verwendet. Hiermit wurden von 75 g 3,5-Dichlor-2,4-difluor-nitrobenzol, die in 200 ml DMSO als Lösungsmittel eingesetzt worden waren, 73,4 g mit Hilfe von n-Hexan als Extraktionsmittel in 6 Stunden extrahiert. Der Extraktor wurde auf 20 ° C temperiert. Pro 100 ml des Gemisches DMSO/3,5-Dichlor-2,4-difluornitrobenzol wurden 150 ml n-Hexan eingesetzt. Das n-Hexan lief bis zum Ende der Extraktion etwa 40 mal um.

Beispiele 2 - 25

Die Durchführung erfolgte analog Beispiel 1 mit ebenfalls 75 g Produkt in 200 ml Lösungsmittel.

Pro 100 ml des Gemisches Lösungsmittel/substituiertes Fluorbenzol wurden 100-200 ml Extraktionsmittel eingesetzt, das etwa 20-80 mal im Extraktor umlief. Das aus der nucleophilen Chlor-Fluor-Austauschreaktion stammende KF/KC1-Salzgemisch blieb im Beispiels 6 während der Extraktion im Reaktionsgemisch und wurde erst nach der Extraktion abgetrennt.

Die unterschiedlichen Lösungsmittel, Extraktionsmittel und Produkte sind in folgender Tabelle wiederge-

geben:

| Beispiel | Produkt | Lösungsmittel | Extraktionsmittel |
|---|---|---|---|
| 2 | 3,5-Dichlor-2,4-difluornitrobenzol | DMSO | Cyclohexan |
| 3 | " | " | Methylcyclohexan |
| 4 | " | " | iso-Dodekan |
| 5 | " | DMF | " |
| 6 | " | " | n-Hexan |
| 7 | " | $CH_3CN$ | " |
| 8 | 3,5-Dichlor-2,4-difluorbenzoylfluorid | Sulfolan | " |
| 9 | 4-Fluor-3-methyl-nitrobenzol | " | " |
| 10 | 4-Fluor-3-trifluormethyl-nitrobenzol | " | " |
| 11 | 4-Fluor-3-chlor-benzonitril | " | " |
| 12 | 4-Fluor-nitrobenzol | " | " |
| 13 | 3-Chlor-4-fluor-nitrobenzol | " | " |
| 14 | " | DMF | " |
| 15 | 4-Fluor-nitrobenzol | DMSO | " |
| 16 | 2-Fluor-nitrobenzol | " | " |
| 17 | " | Sulfolan | " |
| 18 | 2-Fluor-5-chlor-nitrobenzol | DMSO | n-Hexan |
| 19 | " | " | i-Oktan |
| 20 | 2,4-Difluor-5-chlor-nitrobenzol | " | n-Hexan |
| 21 | " | " | Ethyl-cyclohexan |

| | | | |
|---|---|---|---|
| 22 | 2,4-Difluor-nitrobenzol | DMSO | Ethyl-cyclo-hexan |
| 23 | " | " | n-Hexan |
| 24 | 4-Fluor-1,3-dinitrobenzol | " | " |
| 25 | 4-Fluor-3-nitro-benzoesäure-methylester | " | " |

Beispiel 26

Der Ansatz war der gleiche wie in Beispiel 1, die Extraktion wurde jedoch diskontinuierlich durchgeführt. Nach 20 Extraktionsschritten mit jeweils gleichen Volumina DMSO/Hexan hatte man 97 % des Produktes extrahiert.

Beispiel 27

Es wurde wie in Beispiel 11 gearbeitet, jedoch ersetzte man das DMSO durch eine DMSO-Wasser-Mischung, wobei das Produkt in 180 g DMSO gelöst wurde und 20 g $H_2O$ zugesetzt wurden.

Die analytische Verfolgung der Extraktion zeigte, daß schon nach 2 Stunden unter sonst identischen Bedingungen die Extraktion beendet war.

Beispiel 28

130 g 2,3,4,5-Tetrachlor-nitrobenzol (0,5 Mol) wurden mit 75,5 g KF (1,3 Mol) in 130 g DMSO suspendiert und 4 Stunden auf 110° C erwärmt. Nach dem Abkühlen auf Raumtemperatur wurde das KF/KC1-Feststoffgemisch über eine Nutsche abfiltriert und zweimal mit je 30 ml DMSO gewaschen. Die erhaltene DMSO-Lösung wurde kontinuierlich mit kaltem Hexan in einem 300 ml-Rotations-Perforator nach Ludwig zur Flüssig-Flüssig-Extraktion mit spezifisch leichteren Lösungsmitteln (DE-AS 2 221 554, Firma Normag) extrahiert. Der Perforator wurde hierbei von außen mit Wasser gekühlt. Aus der Hexanphase wurde 3,5-Dichlor-2,4-difluor-nitrobenzol in 78 %iger Ausbeute, bezogen auf die theoretische Ausbeute, gewonnen. Die Hexanphase enthielt weiterhin nach gaschromatographischer Bestimmung 10 %, bezogen auf die theoretische Menge, 2,3,4,5-Tetrachlor-fluorbenzol.

Beispiel 29 (nach US 3.294.629; zum Vergleich)

50 g 2,3,4,5-Tetrachlor-nitrobenzol wurden in 150 ml DMSO aufgelöst, 30 g KF wurden hinzugefügt und das Gemisch wurde unter Rühren 7 Stunden auf 100° C erhitzt. Nach dem Abkühlen wurde das Gemisch in etwa 700 ml Wasser gegossen. Hierbei bildete sich eine ölige Schicht, die in etwa 60 ml Chloroform aufgenommen wurde. Die Chloroform-Phase wurde 3 mal mit Wasser gewaschen, getrocknet und unter reduziertem Druck eingeengt. Durch Vakuumdestillation des entstandenen Rückstandes erhielt man 15 g 3,5-Dichlor-2,4-difluor-nitrobenzol als gelbes Öl mit einem Siedepunkt von 71-75,5° C/2 mm Hg. Das entspricht 34 % der theoretischen Ausbeute.

**Patentansprüche**

**1.** Verfahren zur Gewinnung von substituierten Fluorbenzolen aus aprotischen, polaren Lösungsmitteln bzw. solche Lösungsmittel enthaltenden Gemischen, dadurch gekennzeichnet, daß man die substituierten Fluorbenzole mit aliphatischen Extraktionsmitteln aus den Lösungsmitteln/Lösungsmittelgemischen extrahiert und danach die Extraktionsmittel von den substituierten Fluorbenzolen abtrennt oder die substituierten Fluorbenzole in den Extraktionsmitteln einer chemischen Umsetzung unterzieht und die Extraktionsmittel von den Umsetzungsprodukten der substituierten Fluorbenzole abtrennt.

**2.** Verfahren nach Anspruch 1, dadurch gekennzeichnet, daß die substituierten Fluorbenzole solche der allgemeinen Formel

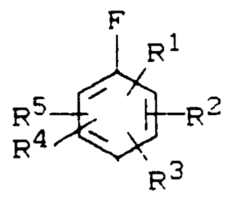

sind, in der

- $R^1$ für eine zum Fluor ortho- oder para-ständige $NO_2$-, CN-, COF- oder $CF_3$-Gruppe steht und für den Fall, daß mindestens drei der Reste $R^2$ bis $R^5$ Chlor oder Brom darstellen, von denen eines zum Fluor ortho-ständig ist, auch das zweite ortho-ständige Chlor oder Brom sein kann,
- $R^2$ Wasserstoff, Halogen, eine $NO_2$-, CN-, COF-, $CF_3$-, $COOR^6$-, $COR^7$-, $SO_2R^7$-, $SO_2$-N-$(R^6)_2$- oder CO-N$(R^6)_2$-Gruppe bedeutet, worin
  $R^6$ Alkyl oder Phenyl und
  $R^7$ Alkyl, Phenyl oder substituiertes Phenyl bedeuten,
- $R^3$ Wasserstoff, Halogen, Alkyl oder eine $NO_2$-Gruppe bedeutet und
- $R^4$ und $R^5$ unabhängig voneinander Wasserstoff oder Halogen bedeuten.

**3.** Verfahren nach Ansprüchen 1 und 2, dadurch gekennzeichnet, daß die substituierten Fluorbenzole gegebenenfalls substituierte Fluor-nitrobenzole der Formel

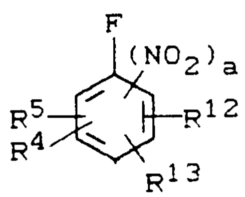

sind, in der

- a die ortho- oder para-Position anzeigt,
- $R^{12}$ und $R^{13}$ unabhängig voneinander Wasserstoff, die $NO_2$- oder $CF_3$-Gruppe oder Halogen bedeuten, wobei $R^{13}$ zusätzlich noch Alkyl bedeuten kann, und
- $R^4$ und $R^5$ unabhängig voneinander Wasserstoff oder Halogen bedeuten.

**4.** Verfahren nach Ansprüchen 1 bis 3, dadurch gekennzeichnet, das die substituierten Fluorbenzole substituierte Fluor-nitrobenzole der Formel

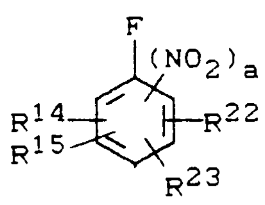

sind, in der

- a die ortho- oder para-Position anzeigt,
- $R^{22}$ und $R^{23}$ unabhängig voneinander die $NO_2$- oder $CF_3$-Gruppe, Fluor oder Chlor bedeuten, wobei $R^{23}$ zusätzlich noch Alkyl bedeuten kann, und
- $R^{14}$ und $R^{15}$ unabhängig voneinander Wasserstoff, Fluor oder Chlor bedeuten.

**5.** Verfahren nach Ansprüchen 1 bis 4, dadurch gekennzeichnet, daß das aprotische, polare Lösungsmittel

Dimethylsulfoxid (DMSO), Dimethylsulfon ($DMSO_2$), Tetramethylensulfon ($TMSO_2$, Sulfolan), Dimethylformamid (DMF), Dimethylacetamid (DMA) oder Acetonitril ist.

**6.** Verfahren nach Ansprüchen 1 bis 5, dadurch gekennzeichnet, daß das aprotische, polare Lösungsmittel DMSO, DMF oder $TMSO_2$ ist.

**7.** Verfahren nach Ansprüchen 1 bis 6, dadurch gekennzeichnet, daß das Extraktionsmittel Hexan, Octan, Isooctan, Dodecan, Isododecan, Leichtbenzin, Ligroin, Lötbenzin, Decalin, Cyclooctan, Cyclohexan, Methyl-cyclohexan, Ethyl-cyclohexan oder Waschbenzin ist.

**8.** Verfahren nach Ansprüchen 1 bis 7, dadurch gekennzeichnet, daß die Extraktion bei einer Temperatur von -20 bis 160° C, bevorzugt von -10 bis 100° C, besonders bevorzugt von 0 bis 60° C, durchgeführt wird.

**9.** Verfahren nach Ansprüchen 1 bis 8, dadurch gekennzeichnet, daß die Extraktion ohne Zugabe von Wasser durchgeführt wird.

**10.** Verfahren nach Ansprüchen 1 bis 9, dadurch gekennzeichnet, daß die Abtrennung des Extraktionsmittels von den substituierten Fluorbenzolen durch Destillation erfolgt.

## Claims

**1.** Process for obtaining substituted fluorobenzenes from aprotic, polar solvents or mixtures containing such solvents, characterised in that the substituted fluorobenzenes are extracted from the solvents/solvent mixtures with aliphatic extracting agents, and the extracting agents are then separated from the substituted fluorobenzenes, or the substituted fluorobenzenes are subjected to a chemical reaction in the extracting agents and the extracting agents are separated from the reaction products of the substituted fluorobenzenes.

**2.** Process according to Claim 1, characterised in that the substituted fluorobenzenes are those of the general formula

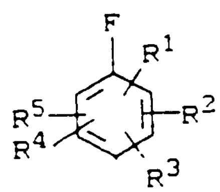

in which

| | |
|---|---|
| $R^1$ | represents an $NO_2$, CN, COF or $CF_3$ group which is in the ortho- or para-position to the fluorine and, in the case where at least three of the radicals $R^2$ to $R^5$ represent chlorine or bromine, of which one is in the ortho-position to the fluorine, the second may also be chlorine or bromine in the ortho-position, |
| $R^2$ | denotes hydrogen, halogen or an $NO_2$, CN, COF, $CF_3$, $COOR^6$, $COR^7$, $SO_2R^7$, $SO_2$-$N(R^6)_2$ or CO-$N(R^6)_2$ group, in which $R^6$ denotes alkyl or phenyl, and $R^7$ denotes alkyl, phenyl or substituted phenyl, |
| $R^3$ | denotes hydrogen, halogen, alkyl or an $NO_2$ group, and |
| $R^4$ and $R^5$, | independently of one another, denote hydrogen or halogen. |

**3.** Process according to Claims 1 and 2, characterised in that the substituted fluorobenzenes are optionally substituted fluoro-nitrobenzenes of the formula

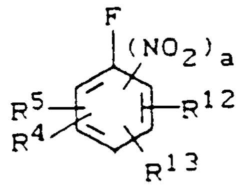

in which

| | |
|---|---|
| a | indicates the ortho- or para-position, |
| $R^{12}$ and $R^{13}$, | independently of one another, denote hydrogen, the $NO_2$ or $CF_3$ group or halogen, where $R^{13}$ may additionally denote alkyl, and |
| $R^4$ and $R^5$, | independently of one another, denote hydrogen or halogen. |

**4.** Process according to Claims 1 to 3, characterised in that the substituted fluorobenzenes are substituted fluoro-nitrobenzenes of the formula

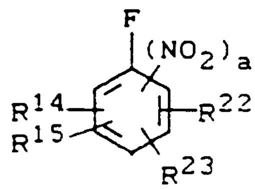

in which

| | |
|---|---|
| a | indicates the ortho- or para-position, |
| $R^{22}$ and $R^{23}$, | independently of one another, denote the $NO_2$ or $CF_3$ group, fluorine or chlorine, where $R^{23}$ may additionally denote alkyl, and |
| $R^{14}$ and $R^{15}$, | independently of one another, denote hydrogen, fluorine or chlorine. |

**5.** Process according to Claims 1 to 4, characterised in that the aprotic, polar solvent is dimethyl sulphoxide (DMSO), dimethyl sulphone ($DMSO_2$), tetramethylene sulphone ($TMSO_2$, sulpholane), dimethylformamide (DMF), dimethylacetamide (DMA) or acetonitrile.

**6.** Process according to Claims 1 to 5, characterised in that the aprotic, polar solvent is DMSO, DMF or $TMSO_2$.

**7.** Process according to Claims 1 to 6, characterised in that the extracting agent is hexane, octane, isooctane, dodecane, isododecane, light petroleum, ligroin, soldering benzine, decalin, cyclooctane, cyclohexane, methyl-cyclohexane, ethyl-cyclohexane or petroleum benzine.

**8.** Process according to Claims 1 to 7, characterised in that the extraction is carried out at a temperature from -20 to 160 ° C, preferably -10 to 100 ° C, particularly preferably 0 to 60 ° C.

**9.** Process according to Claims 1 to 8, characterised in that the extraction is carried out without addition of water.

**10.** Process according to Claims 1 to 9, characterised in that the removal of the extracting agent from the substituted fluorobenzenes is carried out by distillation.

## Revendications

**1.** Procédé pour isoler des fluorobenzènes substitués de solvants polaires aprotoniques ou de mélanges contenant de tels solvants, caractérisé en ce que l'on extrait les fluorobenzènes substitués du solvant/mélange solvant à l'aide d'agents d'extraction aliphatiques puis on sépare les agents d'extraction des fluorobenzènes substitués ou bien on soumet ces derniers, dans les agents d'extraction, à une conversion chimique et on sépare les agents d'extraction des produits de réaction des fluorobenzènes substitués.

**2.** Procédé selon la revendication 1, caractérisé en ce que les fluorobenzènes substitués sont des composés de formule générale :

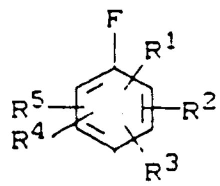

dans laquelle

$R^1$ représente un groupe $NO_2$, CN, COF ou $CF_3$ en position ortho ou para par rapport au fluor, et, dans les cas où au moins trois des symboles $R^2$ à $R^5$ représentent le chlore ou le brome dont un en position ortho par rapport au fluor, le second substituant en position ortho peut également consister en chlore ou brome,

$R^2$ représente l'hydrogène, un halogène, un groupe $NO_2$, CN, COF, $CF_3$, $COOR^6$, $COR^7$ $SO_2R^7$, $SO_2$-$N(R^6)_2$ ou CO-$N(R^6)_2$ dans lesquels
$R^6$ représente un groupe alkyle ou phényle, et
$R^7$ représente un groupe alkyle, phényle ou phényle substitué,

$R^3$ représente l'hydrogène, un halogène, un groupe alkyle ou $NO_2$, et

$R^4$ et $R^5$ représentent chacun, indépendamment l'un de l'autre, l'hydrogène ou un halogène.

**3.** Procédé selon les revendications 1 et 2, caractérisé en ce que les fluorobenzènes substitués sont des fluoronitrobenzènes éventuellement substitués de formule :

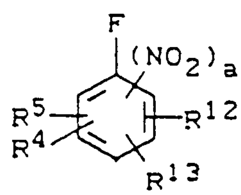

dans laquelle

a indique la position ortho ou para,

$R^{12}$ et $R^{13}$ représentent chacun, indépendamment l'un de l'autre, l'hydrogène, un groupe $NO_2$ ou $CF_3$ ou un halogène, $R^{13}$ pouvant en outre représenter un groupe alkyle,

$R^4$ et $R^5$ représentent chacun, indépendamment l'un de l'autre, l'hydrogène ou un halogène.

**4.** Procédé selon les revendications 1 à 3, caractérisé en ce que les fluorobenzènes substitués sont des fluoronitrobenzènes substitués de formule :

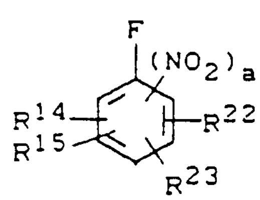

dans laquelle

a indique la position ortho ou para,

$R^{22}$ et $R^{23}$ représentent chacun, indépendamment l'un de l'autre, un groupe $NO_2$ ou $CF_3$, le fluor ou le chlore, $R^{23}$ pouvant également représenter un groupe alkyle,

$R^{14}$ et $R^{15}$ représentent chacun, indépendamment l'un de l'autre, l'hydrogène, le fluor ou le chlore.

**5.** Procédé selon les revendications 1 à 4, caractérisé en ce que le solvant polaire aprotonique est le diméthylsulfoxyde (DMSO), la diméthylsulfone ($DMSO_2$), la tétraméthylène-sulfone ($TMSO_2$, "sulfolan"), le diméthylformamide (DMF), le diméthylacétamide (DMA) ou l'acétonitrile.

**6.** Procédé selon les revendications 1 à 5, caractérisé en ce que le solvant polaire aprotonique est le DMSO, le DMF ou la $TMSO_2$.

**7.** Procédé selon les revendications 1 à 6, caractérisé en ce que l'agent d'extraction est l'hexane, l'octane, l'isooctane, le dodécane, l'isododécane, l'essence minérale légère, l'essence minérale moyenne, l'essence de soudure, la décaline, le cyclooctane, le cyclohexane, le méthylcyclohexane, l'éthylcyclohexane ou l'essence de nettoyage à sec.

**8.** Procédé selon les revendications 1 à 7, caractérisé en ce que l'extraction est effectuée à des températures de -20 à 1600 ° C, de préférence de -10 à 1000 ° C et tout particulièrement de 0 à 60 ° C.

**9.** Procédé selon les revendications 1 à 8, caractérisé en ce que l'extraction est réalisée sans adjonction d'eau.

**10.** Procédé selon les revendications 1 à 9, caractérisé en ce que l'on sépare l'agent d'extraction des fluorobenzènes substitués par distillation.